# EUROPEAN PATENT SPECIFICATION

(11) **EP 4 180 805 B1**
(45) Date of publication and mention of the grant of the patent: **29.01.2025**
(21) Application number: 21383025.0
(22) Date of filing: 12.11.2021
(51) Int. Cl.: G01N 27/48, G01N 33/18

(54) **SCREEN-PRINTED ELECTRODE, MANUFACTURING METHOD THEREOF, ELECTROCHEMICAL SENSOR COMPRISING SAID ELECTRODE FOR DETECTING WATER POLLUTANTS, AND OPERATING METHOD OF SAID SENSOR**
SIEBGEDRUCKTE ELEKTRODE, VERFAHREN ZU IHRER HERSTELLUNG, ELEKTROCHEMISCHER SENSOR MIT DIESER ELEKTRODE ZUM NACHWEIS VON WASSERVERUNREINIGUNGEN UND VERFAHREN ZUM BETRIEB DES SENSORS
ÉLECTRODE FABRIQUÉE PAR SÉRIGRAPHIE, SON PROCÉDÉ DE FABRICATION, CAPTEUR ÉLECTROCHIMIQUE COMPRENANT LADITE ÉLECTRODE POUR DÉTECTER DES POLLUANTS DE L'EAU ET PROCÉDÉ DE FONCTIONNEMENT DUDIT CAPTEUR

(43) Date of publication of application: 17.05.2023
(73) Proprietor: Consejo Superior de Investigaciones Cientificas, 28006 Madrid (ES)
(72) Inventor: DUAN, Wenchao, 08193 Cerdanyola del Vallés (ES); GICH GARCÍA, Martí, 08193 Cerdanyola del Vallés (ES); FERNÁNDEZ SÁNCHEZ, César, 08193 Cerdanyola del Vallés (ES)
(74) Representative: Pons

(56) References cited:
- US-A- 5 126 034
- US-A1- 2010 140 108
- US-A1- 2011 213 229
- US-A1- 2020 191 740
- CARATELLI VERONICA ET AL: "Precision medicine in Alzheimer's disease: An origami paper-based electrochemical device for cholinesterase inhibitors", BIOSENSORS AND BIOELECTRONICS, vol. 165, 29 June 2020 (2020-06-29), Amsterdam , NL, pages 112411, XP055906214, ISSN: 0956-5663, DOI: 10.1016/j.bios.2020.112411
- AKHTAR HAYAT ET AL: "Disposable Screen Printed Electrochemical Sensors: Tools for Environmental Monitoring", SENSORS, vol. 14, no. 6, 13 June 2014 (2014-06-13), pages 10432 - 10453, XP055287538, DOI: 10.3390/s140610432
- HONEYCHURCH K C ET AL: "Screen-printed electrochemical sensors for monitoring metal pollutants", TRAC TRENDS IN ANALYTICAL CHEMISTRY, ELSEVIER, AMSTERDAM, NL, vol. 22, no. 7, 1 July 2003 (2003-07-01), pages 456 - 469, XP004480039, ISSN: 0165-9936, DOI: 10.1016/S0165-9936(03)00703-9
- CARCHI TANYA ET AL: "A Nafion Film Cover to Enhance the Analytical Performance of the CuO/Cu Electrochemical Sensor for Determination of Chemical Oxygen Demand", SENSORS, vol. 19, no. 3, 6 February 2019 (2019-02-06), pages 669, XP055906411, DOI: 10.3390/s19030669

## Description

### FIELD OF THE INVENTION

The present invention lies within the technical field of electrochemical sensors. More specifically, the invention relates to a screen-printed electrode, its manufacturing method, the electrochemical sensor comprising said electrode for detecting water pollutants, and the operating method of said sensor.

### BACKGROUND OF THE INVENTION

Water pollution is an increasing global concern that damages human health, aquatic ecosystems, and economic growth. Globally, 80 percent of municipal wastewater is discharged untreated into water bodies, and industry is responsible for dumping millions of tons of heavy metals, solvents, and other waste effluents each year. Moreover, in most high-income countries and many emerging economies, agricultural activities have become the major factor in the degradation of inland and coastal waters due to the discharge of large quantities of agrochemicals, organic matter, and drug residues (FAO and IWMI, 2017). Therefore, developing simple, cost-effective, and rapid analytical tools for water quality assessment is critical to monitor water pollution and make timely decisions.

For instance, chemical oxygen demand (COD) is a key parameter for the evaluation of water quality [Li et al. Crit. Rev. Anal. Chem. 2018, 48 (1), 47-65]. It is expressed as the amount of oxygen (in milligrams, mg) necessary to decompose all the organic matter contained in one liter (L) of a surface water or wastewater sample. Conventional detection methods evaluate COD by the oxidative degradation of the organic compounds present in a water sample with strong oxidizing agents [Moore et al. Anal. Chem. 1956, 28, 164-167; Korenaga et al. Anal. Chim. Acta 1982, 141, 301-309; Tian et al. Anal. Chim. Acta 1992, 261, 301-305; Lee et al. Electroanalysis 2000, 12, 1334-1338]. However, these methods present many disadvantages, including:
- low sensitivity and precision;
- large sample volume;
- use of hazardous materials, such as silver sulphate, mercury sulphate, and dichromate;
- chloride interference;
- long analysis time, since it requires a time-consuming sample reflux process to achieve complete oxidation (2-4 hours); and
- professional operation.

Numerous efforts have been made to overcome these disadvantages, with electrochemical methods becoming the most promising option for determining not only COD, but also other water contaminants in a rapid, sensitive, operationally simple, and cost-effective manner. An example of said electrochemical methods is the amperometric determination of COD, based on measuring the current during the electrochemical oxidation of the organic species present in the sample performed by strong oxidant hydroxyl radicals produced on the electrode surface [Comninellis. C. Electrochim. Acta 1994, 39, 1857-1862].

For achieving high electrical response signals, the development of highly sensitive electrode materials specific to the analysis of different water contaminants is of paramount importance. In the case of the electrochemical determination of COD, the following electrodes were so far reported:
- activated copper electrode [Silva et al. J. Solid State Electrochem. 2009, 13 (5), 665-669];
- copper electrode modified with copper nanoparticles [Yang et al. Sens. Actuators B Chem. 2011, 153 (1), 78-82];
- glassy carbon electrode coated with nickel (Ni) nanoparticles [Cheng et al. J. Phys. Chem. C 2011, 115 (46), 22845-22850], nickel-copper (NiCu) alloy [Zhou et al. Electrochim. Acta 2012, 74, 165-170], or cobalt oxide (CoO) film [Wang et al. Anal. Chim. Acta 2012, 736, 55-61];
- boron-doped diamond electrode [Yu et al. Electrochem. Commun. 2007, 9 (9), 2280-2285];
- platinum electrode modified with lead dioxide (PbO₂) [Ai et al. Electroanal. 2004, 16 (5), 404-410];
- titanium electrode coated with nano-TiO₂ [Li et al. Electroanalysis. 2006, 18 (10), 1014-1018], Rh₂O₃ [Li et al. Meas. Sci. Technol. 2006, 17 (7), 1995-2000], or TiO₂/PbO₂ [Li et al. Electroanal. 2006, 18 (22), 2251-2256];
- graphite-polystyrene composite electrode containing silver (II) oxide and copper (II) oxide catalysts (AgO-CuO) [Orozco et al. Anal. Chim. Acta. 2008, 607 (2), 176-182]; and,
- carbon nanotube-polystyrene composite containing different inorganic electrocatalysts (Ni, NiCu alloy, CoO, and CuO/AgO nanoparticles) [Gutiérrez-Capitán et al. Anal. Chem. 2015, 87 (4), 2152-2160].

Document US 5 126 034 A discloses an analytical equipment for a bioelectrochemical determination of microorganisms or other cells in a liquid sample, the equipment including a disposable element which comprises a substrate, a plurality of electrode tracks, screen-printed over the substrate, a screen-printed working electrode, a screen-printed pseudo-reference electrode, and a screen-printed auxiliary electrode; an insulating layer arranged over the conductive tracks; and a filtering element, arranged in contact with the electrochemical cell.

However, the manufacture of electrochemical sensors comprising said specific electrodes and, in general, any electrode used for detecting water pollutants, is cumbersome in some cases or cannot be scaled up for mass-production purposes in some others. Most importantly, all these approaches require sample preconditioning before carrying out the analytical measurement, including sample filtration as well as pH and conductivity adjustment. Said sample-preconditioning involves at least two initial steps that are carried out ex-situ, limiting the application of these devices in in-field testing. Therefore, analytical tools for decentralized on-site rapid determination of water contaminants that saves analysis time are in demand.

The present invention proposes a solution to the limitations mentioned above by means of a novel screen-printed electrode that allows the manufacturing of portable, sample-to-result and user-friendly electrochemical sensors for on-site detection of water pollutants.

### BRIEF DESCRIPTION OF THE INVENTION

A first object of the present invention relates to a screen-printed electrode (SPE) for detecting a pollutant in a water sample. The SPE comprises:
- a substrate;
- a plurality of conductive tracks, screen-printed over the substrate;
- an electrochemical cell, connected to said conductive tracks and configured to receive the water sample, said electrochemical cell further comprising:
   - a working electrode, screen-printed over the substrate and configured to enable the electrochemical degradation of the pollutant present in the water sample;
   - a pseudo-reference electrode, screen-printed over the substrate and configured to provide an electric potential against which the potential of the working electrode is set; and,
   - an auxiliary electrode, screen-printed over the substrate and configured to provide a pathway for electric current to flow in the electrochemical cell; and,
- an insulating layer, arranged over the conductive tracks and adapted so as to protect said conductive tracks from a liquid environment.

Within the scope of interpretation of the present invention, the expression "enable the electrochemical degradation" will be understood as making an oxidation or reduction reaction of the pollutant present in the water sample happen or happen more quickly.

According to the invention, the SPE of the invention comprises an electrolyte-impregnated filtering element that is arranged in contact with the electrochemical cell. In addition to filter the water sample received by the electrochemical cell, this electrolyte-impregnated filtering element enables sample preconditioning and subsequent water pollutant detection by the electrochemical cell without any user intervention. Thanks to that, sample pre-processing is not needed, maintaining sample quality as it was collected. Preferably, the filtering element is impregnated with an electrolyte comprising sodium hydroxide, as this alkaline medium favors the electrochemical degradation of the pollutant present in the water sample to be analyzed.

In a preferred embodiment of the invention, the working electrode and, optionally, the auxiliary electrode comprise/s a metal nanoparticle-carbon composite-based ink, preferably, a copper nanoparticle-carbon composite-based ink. More preferably, said copper nanoparticle-carbon composite-based ink comprises a carbon bulk material, a plurality of carbon fibers and a plurality of copper nanoparticles. The carbon bulk material is beneficial for electrochemical applications due to its porosity; the carbon fibers, preferably 5-10 µm in length, enhance conductivity; and the copper nanoparticles act as a catalyst of the electrochemical degradation of the pollutant present in the water sample.

In another preferred embodiment of the invention, the electrolyte-impregnated filtering element is covered with a fixing layer containing a plurality of holes, preferably of a plastic material. Said fixing layer fixes the electrolyte-impregnated filtering element to the electrochemical cell, allowing the water sample to flow through the electrolyte-impregnated filtering element and reach the electrochemical cell through the plurality of holes. More preferably, said fixing layer is covered with a removable protective layer to prevent contamination or electrode degradation before use. Said protective layer can be easily removed just before using the SPE for water pollution analysis.

In another preferred embodiment of the invention, the electrolyte-impregnated filtering element comprises a porous paper material.

A second object of the present invention relates to an electrochemical sensor for measuring chemical oxygen demand in a water sample containing organic matter. The system comprises:
- a screen-printed electrode (SPE) according to any of the embodiments herein described;
- means for applying an electric potential between the working electrode and the pseudo-reference electrode of said SPE; and,
- means for measuring and recording a faradaic current at said working electrode.

Within the scope of interpretation of the present invention, the expression "faradaic current" will be understood as any current generated by the oxidation or reduction of the pollutant present in the water sample.

In a preferred embodiment of the invention, the means for potential application as well as the means for current measurement and recording are comprised in a portable potentiostat powered and controlled by an electronic mobile device.

A third object of the present invention relates to a method of measuring COD in a water sample containing organic matter by means of an electrochemical sensor according to any of the embodiments herein described. Advantageously, said method comprises performing the following steps:
- dispensing the water sample onto the electrolyte-impregnated filtering element;
- applying an electric potential between the working electrode and the pseudo-reference electrode by the means for potential application;
- measuring and recording a faradaic current generated at the working electrode by the means for current measurement and recording; and,
- determining the COD of the water sample from the measured faradaic current.

A fourth object of the present invention relates to a method of fabrication of the SPE herein described. Advantageously, said method comprises performing the following steps in any technically possible order:
- providing a substrate, preferably polymer-based;
- screen-printing a plurality of conductive tracks and a pseudo-reference electrode over the substrate using an electrically conductive material, preferably comprising silver paste;
- screen-printing a working and auxiliary electrode over the substrate using a metal nanoparticle-carbon composite-based ink;
- screen-printing an insulating layer, preferably using a photocurable dielectric paste, and arranging said insulating layer over the conductive tracks;
- providing an electrolyte-impregnated filtering element; and,
- arranging said electrolyte-impregnated filtering element in contact with the electrochemical cell.

Optionally, said method can comprise the step of impregnating the filtering element with an electrolyte.

In a particular embodiment of said method, the working and auxiliary electrodes are screen-printed using a copper nanoparticle-carbon composite-based ink, said ink being prepared as follows:
- preparing an aqueous sample comprising 30-35 wt% resorcinol, 0.2-0.6 wt% sodium carbonate and 64-70 wt% formaldehyde (sample A);
- preparing an aqueous sample comprising copper (II) nitrate hydrate in a concentration comprised between 0.6-0.7 mol/L (sample B);
- mixing samples A and B in a volume ratio A:B comprised between 3:1 and 3.5:1 for a period between 60-75 minutes (sample C);
- dissolving 450 to 600 mg sodium carbonate in three additions of 150 to 200 mg in sample C and stirring for a period between 1-1.5 hours until the pH is comprised between 8 and 9 (sample D);
- heating sample D at a temperature comprised between 55-65°C for a period between 20-24 hours;
- after heating sample D, placing the resulting wet gels in a fume hood at room temperature for at least 2 days;
- carbonizing the resulting copper-carbon composite powder under an argon flux comprised between 80-120 cm³/min at a temperature between 1000-1055°C for a period between 110-130 minutes; and,
- mixing the copper nanoparticle-carbon composite powder with nitrocellulose, preferably 15-20 wt%, prepared in 2-butoxyethyl acetate in a weight molar ratio comprised between 3:1 and 3.5:1 until the resulting paste presented a honey-like texture.

Preferably, any of the methods of fabrication of the SPE herein described can further comprise:
- providing a fixing layer containing a plurality of holes; and,
- fixing the electrolyte-impregnated filtering element to the electrochemical cell by means of said fixing layer.

In this case, the water sample is dispensed onto the electrolyte-impregnated filtering element through the holes of the fixing layer.

A fifth object of the present invention relates to the use of the SPE of the invention for determining COD in surface waters (e.g. lakes and rivers), wastewater, and aqueous hazardous wastes, or for analyzing other water pollutants, such as halide ions, sucralose, and chlorinated disinfection byproducts.

### DESCRIPTION OF THE FIGURES

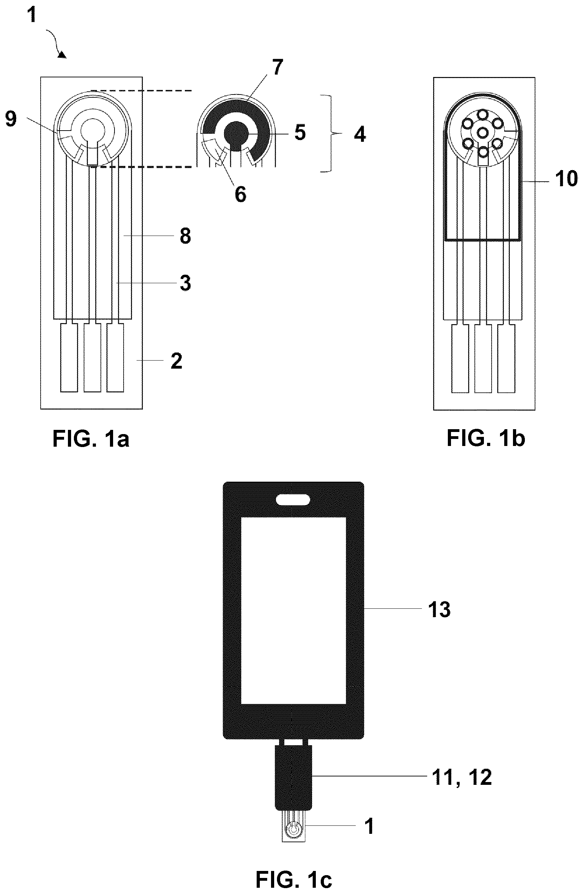

Figure 1a shows the screen-printed electrode (SPE) of the invention according to one of its preferred embodiments. Said electrode is comprised of a substrate, a plurality of conductive tracks, an electrochemical cell, an insulating layer, and a electrolyte-impregnated filtering element, wherein said electrochemical cell further comprises a working electrode, an auxiliary electrode, and a pseudo-reference electrode. Figure 1b shows the SPE of the invention according to another of its preferred embodiments, wherein the electrolyte-impregnated filtering element is covered with a fixing layer containing a plurality of holes. Figure 1c shows the electrochemical sensor of the invention according to one of its preferred embodiments, said sensor comprising the SPE of the invention shown in Figure 1a and a potentiostat powered and controlled by an electronic mobile device.

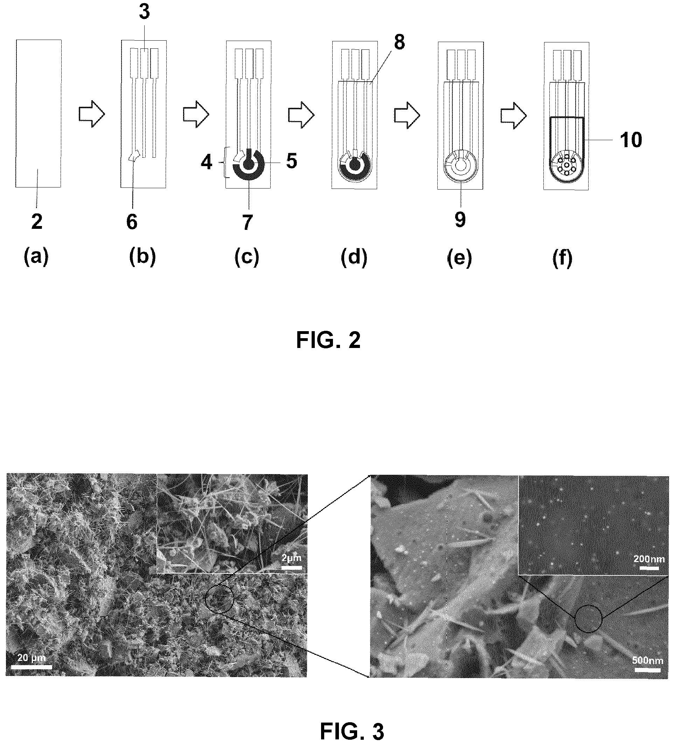

Figure 2 shows the stepwise fabrication of the SPE of the invention according to one of its preferred embodiments: (a) providing a substrate; (b) screen-printing a plurality of conductive tracks and a pseudo-reference electrode over the substrate using an electrically conductive material; (c) screen-printing a working and auxiliary electrode over the substrate using a metal nanoparticle-carbon composite-based ink; (d) screen-printing an insulating layer, preferably using a photocurable dielectric paste, and arranging it over the conductive tracks; (e) providing an electrolyte-impregnated filtering element and arranging it in contact with the electrochemical cell; and (f) providing a fixing layer containing a plurality of holes and fixing the electrolyte-impregnated filtering element to the electrochemical cell by means of said fixing layer.
Figure 3 shows scanning electron microscopy (SEM) images of the copper nanoparticle-carbon composite-based ink used in a preferred embodiment of the method of fabrication of the SPE of the invention.
Figure 4 shows X-ray diffraction patterns of pure carbon and copper nanoparticle-carbon composite-based ink used in a preferred embodiment of the method of fabrication of the SPE of the invention.
Figure 5 shows nitrogen (N₂) adsorption and desorption isotherms of the copper nanoparticle-carbon composite-based ink used in a preferred embodiment of the method of fabrication of the SPE of the invention. Inset shows the pore size distributions determined using the BJH method.
Figure 6 shows SEM images of the copper nanoparticle-carbon composite-based ink used in a preferred embodiment of the method of fabrication of the SPE of the invention grinding at different times using Retsch Mixer Mill MM 400 at the frequency of 15 Hz.
Figure 7 shows the particle size copper nanoparticle-carbon composite-based ink used in a preferred embodiment of the method of fabrication of the SPE of the invention grinding at different times: (A) 20 min, (B) 30 min, (C) 60 min.
Figure 8 shows SEM images of the surface of a screen-printed electrode (SPE) using the copper nanoparticle-carbon composite-based ink used in a preferred embodiment of the method of fabrication of the SPE of the invention. The inset in (A) is the photograph of the SPE and the dimension is 1 cm x 1.1 cm.
Figure 9a shows the chronoamperometric responses of the SPE of the invention in one of its preferred embodiments; that is, with a filtering element but not loaded with NaOH. Figure 9b shows the corresponding calibration curve. Values are the mean of three consecutive measurements and the standard deviation is drawn as error bars.
Figure 10a shows the chronoamperometric responses of the SPE of the invention in one of its preferred embodiments; that is; with a filtering element loaded with NaOH. Figure 10b shows the corresponding calibration curve. Values are the mean of three consecutive measurements and the standard deviation is drawn as error bars.

### NUMERICAL REFERENCES USED IN THE DRAWINGS

In order to provide a better understanding of the technical features of the invention, the referred Figures 1-10 are accompanied by a series of numerical references which, with an illustrative and non-limiting character, are hereby represented:

| | |
|---|---|
| 1 | Screen-printed electrode |
| 2 | Substrate |
| 3 | Conductive track |
| 4 | Electrochemical cell |
| 5 | Working electrode |
| 6 | Pseudo-reference electrode |
| 7 | Auxiliary electrode |
| 8 | Insulating layer |
| 9 | Electrolyte-impregnated filtering element |
| 10 | Fixing layer |
| 11 | Means for potential application |
| 12 | Means for current measurement and recording |
| 13 | Electronic mobile device |

### DETAILED DESCRIPTION OF THE INVENTION

As described in the preceding paragraphs, one object of the present invention relates to a screen-printed electrode (SPE) (1) for detecting a pollutant in a water sample. In the example of the SPE chosen to illustrate the present invention (Figure 1a), the electrode comprises:
- a polymer substrate (2);
- a plurality of conductive tracks (3), screen-printed over the substrate (2);
- an electrochemical cell (4), connected to said conductive tracks (2) and configured to receive the water sample, said electrochemical cell (4) further comprising:
   - a working electrode (5), screen-printed over the substrate (2) and configured to enable the electrochemical degradation of the pollutant present in the water sample;
   - a pseudo-reference electrode (6), screen-printed over the substrate (2) and configured to provide an electric potential against which the potential of the working electrode (5) is set;
   - an auxiliary electrode (7), screen-printed over the substrate (2) and configured to provide a pathway for electric current to flow in the electrochemical cell (4); and,
   - an insulating layer (8), arranged over the conductive tracks (3) and adapted so as to protect said conductive tracks (3) from a liquid environment.

Advantageously, the SPE (1) further comprises an electrolyte-impregnated filtering element (9), preferably of a porous paper material, that is arranged in contact with the electrochemical cell (4). This electrolyte-impregnated filtering element (9) filters and preconditions the water sample received by the electrochemical cell (4), thus saving time in pre-processing samples while avoiding any possible contamination thereof by user manipulation. Preferably, the filtering element (9) is impregnated with an electrolyte comprising sodium hydroxide, as this alkaline medium favorsthe electrochemical degradation of the pollutant present in the water sample.

The electrolyte-impregnated filtering element (9) can be covered with a fixing layer (10) containing a plurality of holes, preferably of a plastic material. Said fixing layer (10) fixes the filtering element (9) to the electrochemical cell (4), allowing the water sample to flow through the filtering element (9) and reach the electrochemical cell (4) through the plurality of holes (Fig. 1b). Optionally, said fixing layer (10) can be covered with a removable protective layer to prevent contamination or electrode degradation before use. Said protective layer can be easily removed just before using the SPE for water pollution analysis.

The working electrode (5) and, optionally, the auxiliary electrode (7) comprise/s a metal nanoparticle-carbon composite-based ink, preferably, a copper nanoparticle-carbon composite-based ink.

A second object of the present invention relates to an electrochemical sensor for measuring chemical oxygen demand in a water sample containing organic matter. The system comprises:
- a SPE (1) according to any of the embodiments herein described;
- means (11) for applying an electric potential between the working electrode (5) and the pseudo-reference electrode (6) of said SPE (1); and,
- means (12) for measuring and recording a faradaic current at said working electrode (5).

Said means (11, 12) for potential application and for current measurement and recording are preferably comprised in a portable potentiostat powered and controlled by an electronic mobile device (13) (Fig.1c).

A third object of the present invention relates to a method of measuring chemical oxygen demand in a water sample containing organic matter by means of an electrochemical sensor according to any of the embodiments herein described. Advantageously, said method comprises performing the following steps:
- dispensing the water sample onto the electrolyte-impregnated filtering element (9);
- applying an electric potential between the working electrode (5) and the pseudo-reference electrode (6) by the means (11) for potential application;
- measuring and recording the faradaic current generated at the working electrode (7) by the means (12) for current measurement and recording; and,
- determining the COD of the water sample from the measured faradaic current.

A fourth object of the present invention relates to a method of fabrication of the SPE (1) herein described (see Figure 2). Said method comprises performing the following steps in any technically possible order:
- providing a substrate (2), preferably polymer-based;
- screen-printing a plurality of conductive tracks (3) and a pseudo-reference electrode (6) over the substrate (2) using an electrically conductive material, preferably comprising silver paste;
- screen-printing a working (5) and auxiliary (7) electrode over the substrate (2) using a metal nanoparticle-carbon composite-based ink, preferably comprising a copper nanoparticle-carbon composite;
- screen-printing an insulating layer (8) using a photocurable dielectric paste, and arranging said insulating layer (8) over the conductive tracks (3);
- providing an electrolyte-impregnated filtering element (9); and,
- arranging said electrolyte-impregnated filtering element (9) in contact with the electrochemical cell (4).

Said copper nanoparticle-carbon composite-based ink is prepared as follows:
- preparing an aqueous sample comprising 30-35 wt% resorcinol, 0.2-0.6 wt% sodium carbonate and 64-70 wt% formaldehyde (sample A);
- preparing an aqueous sample comprising copper (II) nitrate hydrate in a concentration comprised between 0.6-0.7 mol/L (sample B);
- mixing samples A and B in a volume ratio A:B comprised between 3:1 and 3.5:1 for a period between 60-75 minutes (sample C);
- dissolving 450 to 600 mg sodium carbonate in three additions of 150 to 200 mg in sample C and stirring for a period between 1-1.5 hours until the pH is comprised between 8 and 9 (sample D);
- heating sample D at a temperature comprised between 55-65 °C for a period between 20-24 hours;
- after heating sample D, placing the resulting wet gels in a fume hood at room temperature for at least 2 days;
- carbonizing the resulting copper-carbon composite powder under an argon flux of 80-120 cm³/min at a temperature between 1000-1055 °C for a period between 110-130 minutes; and,
- mixing the copper nanoparticle-carbon composite powder with 15-20 wt% nitrocellulose prepared in 2-butoxyethyl acetate in a weight molar ratio comprised between 3:1 and 3.5:1 until the resulting paste presented a honey-like texture.

Optionally, the method of fabrication of the invention can comprise the step of impregnating the filtering element (9) with an electrolyte.

Preferably, any of the methods of fabrication described above can further comprise:
- providing a fixing layer (10) containing a plurality of holes; and,
- fixing the electrolyte-impregnated filtering element (9) to the electrochemical cell (4) by means of said fixing layer (10).

In this case, the water sample is dispensed onto the electrolyte-impregnated filtering element (9) through the holes of the fixing layer (10).

A fifth object of the present invention relates to the use of the SPE (1) of the invention for determining chemical oxygen demand in surface water (e.g. lakes and rivers), wastewater, and aqueous hazardous wastes, or for analyzing other water pollutants, such as halide ions, sucralose, and chlorinated disinfection byproducts.

### Characterization of microstructure and properties of copper nanoparticle-carbon composite-based ink

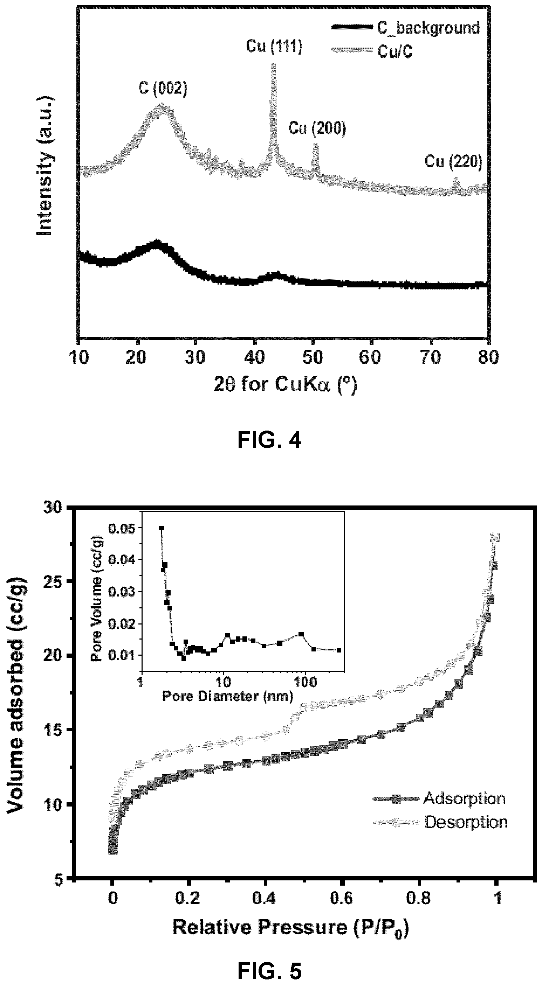

Figure 3 shows scanning electron microscopy images of the copper nanoparticle-carbon composite-based ink used in a preferred embodiment of the method of fabrication of the SPE of the invention, which is made of carbon bulk, carbon fibers and copper nanoparticles. Its crystal structure was examined by X-ray diffraction (XRD) against pure carbon, depicted in grey and black, respectively, in Figure 4. The broad bumps located around 2θ values of 23.5° and 43.5° are characteristic of pure amorphous C. The diffraction peaks observed are characteristic of face-centered cubic (fcc) crystalline copper, corresponding to the planes (111), (200) and (220), at 2θ values of ca. 43.2°, 50.4° and 74.1°, respectively, which demonstrate that the synthesized copper nanoparticle-carbon composite-based ink contains metallic copper nanoparticles.

The porosity of the copper nanoparticle-carbon composite-based ink was measured by nitrogen adsorption and desorption isotherms (Figure 5). According to the Brunauer-Emmett-Teller (BET) model, the surface areas were calculated to be 45 m²/g. The adsorption uptake at low nitrogen relative pressures (P/Po=0.0-0.1) indicates that the Cu/C material presents a lot of micropores (diameter < 2nm). The slope of the isotherms at intermediate relative pressures (0.3<P/P₀<0.8) and the increase in the adsorbed volume at high relative pressures (0.9<P/P₀<1.0) reveal the existence of mesopores (2-50 nm) and macropores (50-7500 nm), respectively. The total pore volume calculation was 0.043 cm³/g based on the N₂ amount adsorbed at a relative pressure P/P₀ of ca. 0.995. The BJH pore size distribution curve acquired from the adsorption isotherm confirmed the predominant diameters in the micropore and mesopore region with the coexistence of a small number of macropores.

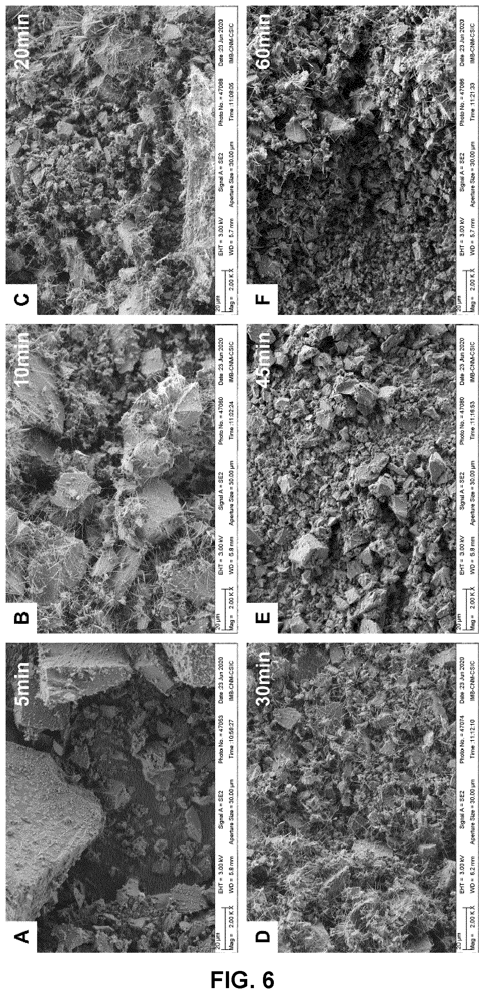

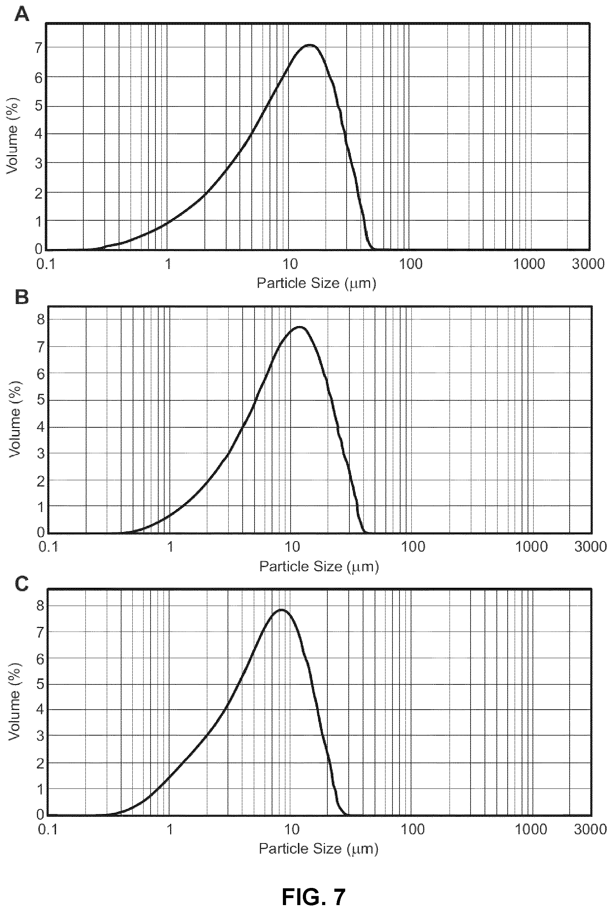

Figure 6 shows SEM images of the copper nanoparticle-carbon composite-based ink at different grinding times showing that, as the grinding time increases, the size of the carbon particles decreases. The Cu/C composite materials with grinding times of 20 min, 30 min and 60 min were selected to study their particle size distribution (Figure 7) and the conductivity of the inks made with them after painting the ink on an insulating polyethylene terephthalate (PET) substrate and letting it dry (Table 1). The best conductivity was obtained when the grinding time is 30 min and the average particle size was 10.86 µm. Therefore, this particle size was used for the preparation of the ink for the screen-printed electrodes.

**Table 1. Values of the conductivity of inks prepared with Cu/C of different particle sizes.**

| Grinding time (min) | Particle size (µm) | Conductivity (S/cm) |
|---|---|---|
| 20 | 12.62 | 0.69±0.27 |
| 30 | 10.86 | 1.32±0.26 |
| 60 | 7.44 | 0.39±0.06 |

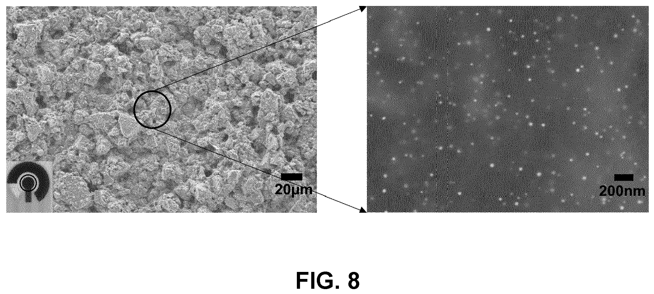

Figure 8 shows the SEM images of the rough surface of the screen-printed working electrode made of Cu/C nanocomposite. It is like the surface of any carbon (graphite) screen-printed electrode. A higher magnification SEM image reveals both the carbon and Cu particle components dispersed in the ink. Energy-Dispersive X-Ray (EDX) analysis of the electrode surface indicates the presence of 4.3 wt.% copper element in the working electrode.

### Example of the electrochemical performance of the SPE-based sensor of the invention for measuring COD in a water sample

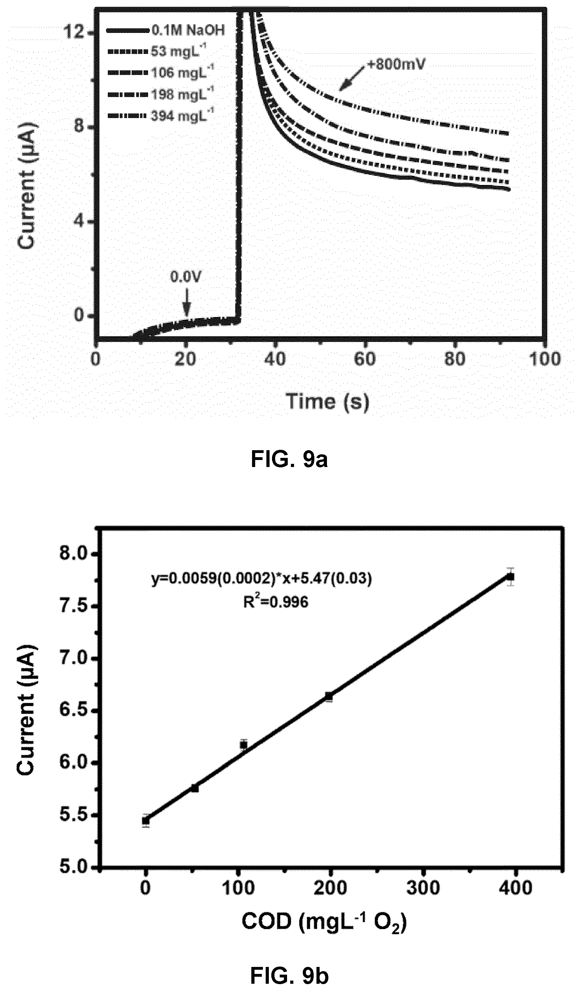

Figure 9 shows the chronoamperograms and the calibration curve of the SPE of the invention with the filtering element (9) not loaded with NaOH, wherein the working electrode (5) and the auxiliary electrode (7) comprise the copper nanoparticle-carbon composite-based ink whose characterization has been previously shown. In the chronoamperometric measurements, a potential of 0.0 mV vs. silver pseudo-reference electrode (6) was initially set for 30 s by the means (11) for potential application, at which no redox reactions occurred and the current tended to zero. Then the potential was shifted to +800.0 mV vs. silver pseudo-reference electrode (6), at which the Cu nanoparticles catalyze the electrocatalytic oxidation of organic matter and the anodic current was recorded for 60 s by the means (12) for current measurement and recording. The total time for one measurement is 690 s (600 s for allowing the sample flow to reach the electrochemical cell and 90s for electrochemical analysis). Figure 9a displays the corresponding chronoamperometric signals for different concentrations of glucose, used as an organic standard analyte. Based on these chronoamperograms, the value of the current recorded at 90 s time was chosen as the analytical signal. The signal increases linearly with the glucose concentration. The corresponding calibration curve is presented in Figure 9b, and a linear range from 0 to 394 mg/L was obtained. The slope of the calibration curve was 5.9±0.2 nA·L/mg. The estimated limit of detection (LOD) is 24.4 mg/L. Water samples from effluents of wastewater treatment plants cannot show organic matter concentrations above the legal limit of COD, set to 125 mg/L, or a minimum 75% reduction with relation to the organic load of the influent. Considering that, the SPE-based sensor of the invention with the filtering element (9) not loaded with NaOH results in a promising tool for the analysis of COD in wastewater.

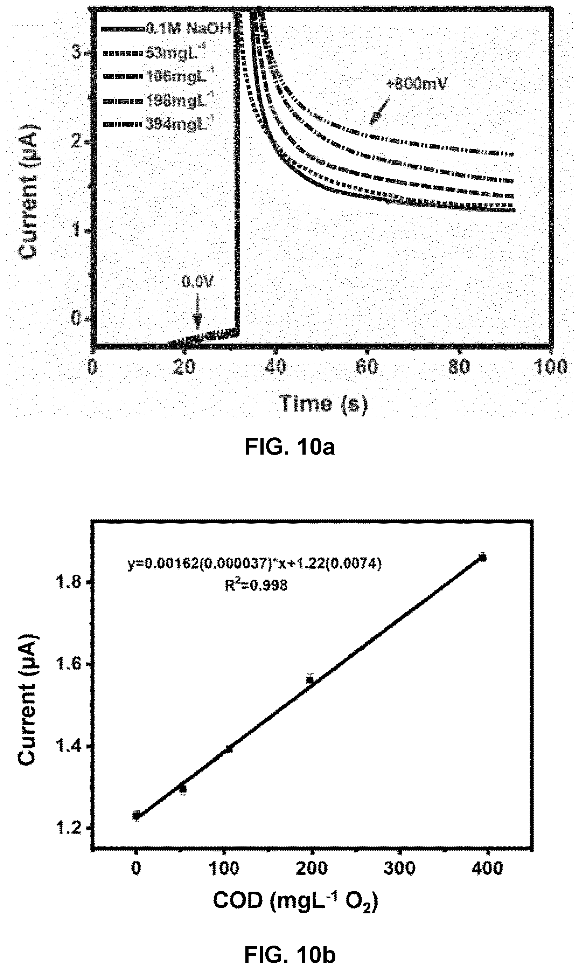

Figure 10 shows the chronoamperogram and the calibration curve of the SPE of the invention with the filtering element (9) loaded with NaOH. As before, for conducting the chronoamperometric analysis a potential of +800.0 mV vs silver pseudo-reference electrode (6) was set by the means (11) for potential application, and the corresponding calibration curve was plotted by the means (12) for current measurement and recording. The current value at the 90 s time was used as the analytical signal. Then a linear range from 0 to 394 mg/L was obtained (Figure 10b), and the slope of the calibration curve was 1.62±0.04 nA·L/mg. The estimated limit of detection (LOD) is 25.99 mg/L. As mentioned above, the wastewater treatment plants have a legal COD limit in the effluents set to 125 mg/L. Thus, the SPE-based sensor with the filtering element (9) impregnated with NaOH can be applied for the analysis of COD in wastewater. Besides, said sensor is simple, convenient, and easy to operate, so it can be implemented to measure this parameter in real water samples, as shown below.

### Analysis of wastewater samples using the SPE-based sensor of the invention

Three real samples from a wastewater treatment plant were collected and analyzed with the screen-printed electrode of the invention, having the filtering element (9) impregnated (SPE_Cu/C_filter_{NaOH}) or not (SPE_Cu/C filter) with NaOH and compared with the performance of the same SPE but without a filtering element (SPE_Cu/C). As can be seen in Table 2, the values recorded with the three approaches were quite similar, which shows that the filtering element (9) successfully performed for filtering and preconditioning the sample before the measurement. Moreover, the values recorded with the sensors are, within the error limits, consistent with the values obtained from the standard dichromate method produced by a certified laboratory. Overall, the performance of the SPE-based sensor of the invention was highly suitable to determine the COD in real water samples.

**Table 2. COD analysis of real water samples using SPEs and standard dichromate method.**

| **Electrodes** | **Sample 1 Effluent (mgL⁻¹O₂)** | **Sample 2 Primary treatment (mgL⁻¹O₂)** | **Sample 3 Initial process (mgL⁻¹O₂)** | **Soaking time** | **Pre-filtered** |
|---|---|---|---|---|---|
| SPE_Cu/C | 38.9±4.6 | 98.8±2.7 | 220±10.8 | 10min | Yes |
| SPE_Cu/C_filter | 39.6±3.8 | 96.4±6.6 | 218±11.2 | 10min | Yes |
| SPE_Cu/C_filter | 41.2±4.2 | 99.2±4.8 | 222.4±13.6 | 10min | No |
| SPE_Cu/C_filter_{NaOH} | 40.1±5.4 | 97.8±6.7 | 221.1±14.5 | 10min | No |
| SPE_Cu/C_filter_{NaOH} | 42.1±6.2 | 100.3±2.1 | 228.5±18.4 | 10min | No |
| Dichromate method | 37.2±7.8 | 84.9±17.8 | 210±25 | | Yes |

## Claims

1. A screen-printed electrode (1) for detecting a pollutant in a water sample comprising:
- a substrate (2);
- a plurality of conductive tracks (3), screen-printed over the substrate (2);
- an electrochemical cell (4), connected to said conductive tracks (3) and configured to receive the water sample, said electrochemical cell (4) further comprising:
- a working electrode (5), screen-printed over the substrate (2) and configured to enable the electrochemical degradation of the pollutant present in the water sample;
- a pseudo-reference electrode (6), screen-printed over the substrate (2) and configured to provide a stable electric potential against which the potential of the working electrode (5) is set; and,
- an auxiliary electrode (7), screen-printed over the substrate (2) and configured to provide a pathway for an electric current to flow in the electrochemical cell (4);
- an insulating layer (8), arranged over the conductive tracks (3) and adapted so as to protect said conductive tracks (3) from a liquid environment; and
- a filtering element (9), arranged in contact with the electrochemical cell (4) and configured to filter the water sample received by the electrochemical cell (4);
and **characterized in that** the filtering element (9) is impregnated with an electrolyte.

2. A screen-printed electrode according to the preceding claim, wherein the working electrode (5) and, optionally, the auxiliary electrode (7) comprise/s a metal nanoparticle-carbon composite-based ink.

3. A screen-printed electrode according to the preceding claim, wherein the metal nanoparticle-carbon composite-based ink comprises a carbon bulk material, a plurality of carbon fibers and a plurality of copper nanoparticles.

4. A screen-printed electrode according to any of the preceding claims, wherein the electrolyte-impregnated filtering element (9):
- comprises a porous paper material; and/or
- is covered with a plastic fixing layer (10) containing a plurality of holes

5. A screen-printed electrode according to any of the preceding claims, wherein the electrolyte comprises sodium hydroxide.

6. An electrochemical sensor for measuring chemical oxygen demand in a water sample containing organic matter, **characterized in that** said electrochemical system comprises:
- a screen-printed electrode (1) according to any of the preceding claims;
- means (11) for applying an electric potential between the working electrode (5) and the pseudo-reference electrode (6) of said screen-printed electrode (1); and,
- means (12) for measuring and recording a faradaic current at said working electrode (5).

7. An electrochemical sensor according to the preceding claim, wherein the means (11) for potential application and the means (12) for current measurement and recording are comprised in a portable potentiostat powered and controlled by an electronic mobile device (13).

8. Method of measuring chemical oxygen demand in a water sample containing organic matter by means of the electrochemical sensor according to any of claims 6-7, **characterized in that** said method comprises performing the following steps:
- dispensing the water sample onto the electrolyte-impregnated filtering element (9);
- applying an electric potential between the working electrode (5) and the pseudo-auxiliary reference electrode (6) by the means (11) for potential application;
- measuring and recording a faradaic current generated at the working electrode (5) by the means (12) for current measurement and recording; and,
- determining the COD of the water sample from the measured faradaic current.

9. Method of fabrication of a screen-printed electrode (1) according to claim 1, **characterized in that** said method comprises performing the following steps in any technically possible order:
- providing a substrate (2);
- screen-printing a plurality of conductive tracks (3) and a pseudo-reference electrode (6) over the substrate (2) using an electrically conductive material;
- screen-printing a working (5) and auxiliary (7) electrode over the substrate (2) using a metal nanoparticle-carbon composite-based ink;
- screen-printing an insulating layer (8) using a photocurable dielectric paste, and arranging said insulating layer (8) over the conductive tracks (3);
- providing an electrolyte-impregnated filtering element (9); and,
- arranging said electrolyte-impregnated filtering element (9) in contact with the electrochemical cell (4).

10. Method of fabrication of a screen-printed electrode (1) according to the preceding claim, further comprising the step of impregnating the filtering element (9) with an electrolyte..

11. Method of fabrication of a screen-printed electrode (1) according to any of claims 9-10, wherein said method further comprises:
- preparing a copper nanoparticle-carbon nanocomposite-based ink; and,
- screen-printing the working (5) and the auxiliary (7) electrode over the substrate (1) using said copper nanoparticle-carbon nanocomposite-based ink.

12. Method of fabrication of a screen-printed electrode (1) according to the preceding claim, wherein the step of preparing a copper nanoparticle-carbon nanocomposite-based ink further comprises:
- preparing an aqueous sample comprising 30-35 wt% resorcinol, 0.2-0.6 wt% sodium carbonate and 64-70 wt% formaldehyde (sample A);
- preparing an aqueous sample comprising copper (II) nitrate hydrate in a concentration between 0.6-0.7 mol/L (sample B);
- mixing samples, A and B in a volume ratio A:B comprised between 3:1 and 3.5:1 for a period between 60-75 minutes (sample C);
- dissolving 450 to 600 mg sodium carbonate in three additions of 150 to 200 mg in sample C and stirring for 1-1.5 hour until the pH is comprised between 8 and 9 (sample D);
- heating sample D at a temperature between 55-65°C for a period between 20 and 24 hours, resulting a plurality of wet gels:
- placing the wet gels in a fume hood at room temperature for at least 2 days;
- carbonizing the resulting copper nanoparticle-carbon nanocomposite powder under an argon flux comprised between 80-120 cm³/min at a temperature between 1000-1055°C for a period between 110-130 minutes; and,
- mixing the copper nanoparticle-carbon composite powder with 15-20%wt nitrocellulose prepared in 2-butoxyethyl acetate in a weight molar ratio comprised between 3:1 and 3.5:1 until the resulting paste presented a honey-like texture.

13. Method of fabrication of a screen-printed electrode (1) according to any of claims 9-12, further comprising:
- providing a plastic fixing layer (10) containing a plurality of holes; and
- fixing the electrolyte-impregnated filtering element (9) to the electrochemical cell (4) by means of said plastic fixing layer (10).

14. Use of the screen-printed electrode (1) according to any of claims 1-5 for:
- determining chemical oxygen demand in surface water, wastewater, and aqueous hazardous wastes; or,
- detecting halide ions, sucralose, or chlorinated disinfection byproducts.

## Patentansprüche

1. Dickfilmelektrode (1) zum Nachweisen eines Schadstoffs in einer Wasserprobe, umfassend:
- ein Substrat (2);
- eine Vielzahl von Leiterbahnen (3), die im Siebdruckverfahren auf das Substrat gedruckt (2) sind;
- eine elektrochemische Zelle (4), die mit den Leiterbahnen (3) verbunden und für die Aufnahme der Wasserprobe ausgelegt ist, wobei die elektrochemische Zelle (4) ferner umfasst:
- eine Arbeitselektrode (5), die im Siebdruckverfahren auf das Substrat (2) gedruckt und so gestaltet ist, dass sie den elektrochemischen Abbau des in der Wasserprobe vorhandenen Schadstoffs ermöglicht;
- eine Pseudo-Referenzelektrode (6), die im Siebdruckverfahren auf das Substrat (2) gedruckt und so gestaltet ist, dass sie ein stabiles elektrisches Potenzial liefert, gegen das das Potenzial der Arbeitselektrode (5) eingestellt wird; und
- eine Hilfselektrode (7), die im Siebdruckverfahren auf das Substrat (2) gedruckt und so gestaltet ist, dass sie einen Weg für den Fluss eines elektrischen Stroms in der elektrochemischen Zelle (4) bereitstellt;
- eine Isolierschicht (8), die über den Leiterbahnen (3) angeordnet ist und so ausgelegt ist, dass sie die Leiterbahnen (3) vor einer flüssigen Umgebung schützt; und
- ein Filterelement (9), das in Kontakt mit der elektrochemischen Zelle (4) angeordnet und so ausgelegt ist, dass es die von der elektrochemischen Zelle (4) aufgenommene Wasserprobe filtert;
und **dadurch gekennzeichnet, dass** das Filterelement (9) mit einem Elektrolyten imprägniert ist.

2. Dickfilmelektrode nach dem vorhergehenden Anspruch, wobei die Arbeitselektrode (5) und, optional, die Hilfselektrode (7) eine Tinte auf der Basis von Metallnanopartikeln und Kohlenstoffkomposit umfasst/umfassen.

3. Dickfilmelektrode nach dem vorhergehenden Anspruch, wobei die Tinte auf der Basis von Metallnanopartikeln und Kohlenstoffkomposit ein Kohlenstoff-Volumenmaterial, eine Vielzahl von Kohlenstofffasern und eine Vielzahl von Kupfer-Nanopartikeln umfasst.

4. Dickfilmelektrode nach einem der vorhergehenden Ansprüche, wobei das mit Elektrolyt imprägnierte Filterelement (9):
- ein poröses Papiermaterial umfasst; und/oder
- mit einer Befestigungsschicht aus Kunststoff (10) überzogen ist, die eine Vielzahl von Löchern enthält.

5. Dickfilmelektrode nach einem der vorhergehenden Ansprüche, wobei der Elektrolyt Natriumhydroxid umfasst.

6. Elektrochemischer Sensor zum Messen des chemischen Sauerstoffbedarfs in einer Wasserprobe, die organische Stoffe enthält, **dadurch gekennzeichnet, dass** das elektrochemische System Folgendes umfasst:
- eine Dickfilmelektrode (1) nach einem der vorhergehenden Ansprüche;
- Mittel (11) zum Anlegen eines elektrischen Potenzials zwischen der Arbeitselektrode (5) und der Pseudo-Referenzelektrode (6) der Dickfilmelektrode (1); und
- Mittel (12) zum Messen und Aufzeichnen eines Faraday-Stroms an der Arbeitselektrode (5).

7. Elektrochemischer Sensor nach dem vorhergehenden Anspruch, wobei die Mittel (11) zum Anlegen des Potenzials und die Mittel (12) zur Strommessung und -aufzeichnung in einem tragbaren Potentiostaten enthalten sind, der von einer elektronischen mobilen Vorrichtung (13) betrieben und gesteuert wird.

8. Verfahren zum Messen des chemischen Sauerstoffbedarfs in einer Wasserprobe, die organisches Material enthält, mithilfe des elektrochemischen Sensors nach einem der Ansprüche 6-7, **dadurch gekennzeichnet, dass** das Verfahren das Durchführen der folgenden Schritte umfasst:
- Abgeben der Wasserprobe auf das mit Elektrolyt imprägnierte Filterelement (9);
- Anlegen eines elektrischen Potenzials zwischen der Arbeitselektrode (5) und der Pseudo-Hilfsreferenzelektrode (6) durch die Mittel (11) zur Potenzialanlegung;
- Messen und Aufzeichnen eines an der Arbeitselektrode (5) erzeugten Faraday-Stroms durch die Mittel (12) zur Strommessung und - aufzeichnung; und
- Bestimmen des CSB der Wasserprobe anhand des gemessenen Faraday-Stroms.

9. Verfahren zur Herstellung einer Dickfilmelektrode (1) nach Anspruch 1, **dadurch gekennzeichnet, dass** das Verfahren das Durchführen der folgenden Schritte in einer beliebigen technisch möglichen Reihenfolge umfasst:
- Bereitstellen eines Substrats (2);
- Drucken einer Vielzahl von Leiterbahnen (3) und einer Pseudo-Referenzelektrode (6) im Siebdruckverfahren auf das Substrat (2) unter Verwendung eines elektrisch leitenden Materials;
- Drucken einer Arbeits- (5) und einer Hilfselektrode (7) im Siebdruckverfahren auf das Substrat (2) unter Verwendung einer Tinte auf der Basis von Metallnanopartikeln und Kohlenstoffkomposit;
- Drucken einer Isolierschicht (8) im Siebdruckverfahren unter Verwendung einer fotohärtbaren dielektrischen Paste und Anordnen der Isolierschicht (8) über den Leiterbahnen (3);
- Bereitstellen eines mit Elektrolyt imprägnierten Filterelements (9); und
- Anordnen des mit Elektrolyt imprägnierten Filterelements (9) in Kontakt mit der elektrochemischen Zelle (4).

10. Verfahren zur Herstellung einer Dickfilmelektrode (1) nach dem vorhergehenden Anspruch, das ferner den Schritt des Imprägnierens des Filterelements (9) mit einem Elektrolyten umfasst.

11. Verfahren zur Herstellung einer Dickfilmelektrode (1) nach einem der Ansprüche 9-10, wobei das Verfahren ferner Folgendes umfasst:
- Herstellen einer Tinte auf der Basis von Kupfer-Nanopartikeln und Kohlenstoff-Nanokomposit; und
- Drucken der Arbeits- (5) und der Hilfselektrode (7) im Siebdruckverfahren auf das Substrat (1) unter Verwendung der Tinte auf der Basis von Kupfer-Nanopartikeln und Kohlenstoff-Nanokomposit.

12. Verfahren zur Herstellung einer Dickfilmelektrode (1) nach dem vorhergehenden Anspruch, wobei der Schritt des Herstellens einer Tinte auf der Basis von Kupfer-Nanopartikeln und Kohlenstoff-Nanokomposit ferner umfasst:
- Herstellen einer wässrigen Probe mit 30-35 Gew.-% Resorcin, 0,2-0,6 Gew.-% Natriumcarbonat und 64-70 Gew.-% Formaldehyd (Probe A);
- Herstellen einer wässrigen Probe mit Kupfer(II)-nitrat-Hydrat in einer Konzentration zwischen 0,6-0,7 mol/l (Probe B);
- Mischen der Proben A und B in einem Volumenverhältnis A:B zwischen 3:1 und 3,5:1 über einen Zeitraum von 60-75 Minuten (Probe C);
- Auflösen von 450 bis 600 mg Natriumcarbonat in drei Zugaben von 150 bis 200 mg in Probe C und Rühren über 1-1,5 Stunden, bis der pH-Wert zwischen 8 und 9 liegt (Probe D);
- Erhitzen der Probe D auf eine Temperatur zwischen 55-65 °C über einen Zeitraum zwischen 20 und 24 Stunden, wodurch eine Vielzahl von nassen Gelen entsteht:
- Aufbewahren der nassen Gele in einem Abzug bei Raumtemperatur über mindestens 2 Tage;
- Karbonisieren des resultierenden Kupfer-Nanopartikel-Kohlenstoff-Nanokomposit-Pulvers unter einem Argonfluss zwischen 80-120 cm³/min bei einer Temperatur zwischen 1000-1055 °C über einen Zeitraum zwischen 110-130 Minuten; und
- Mischen des Kupfer-Nanopartikel-Kohlenstoffkomposit-Pulvers mit 15-20 Gew.-% Nitrocellulose, die in 2-Butoxyethylacetat hergestellt wurde, in einem molaren Verhältnis zwischen 3:1 und 3,5:1, bis die resultierende Paste eine honigartige Textur aufweist.

13. Verfahren zur Herstellung einer Dickfilmelektrode (1) nach einem der Ansprüche 9-12, ferner umfassend:
- Bereitstellen einer Befestigungsschicht aus Kunststoff (10), die eine Vielzahl von Löchern enthält; und
- Befestigen des mit Elektrolyt imprägnierten Filterelements (9) an der elektrochemischen Zelle (4) mithilfe der Befestigungsschicht aus Kunststoff (10).

14. Verwendung der Dickfilmelektrode (1) nach einem der Ansprüche 1-5 zum:
- Bestimmen des chemischen Sauerstoffbedarfs in Oberflächengewässern, Abwässern und wässrigen gefährlichen Abfällen; oder
- Nachweisen von Halogenidionen, Sucralose oder chlorierten Desinfektionsnebenprodukten.

## Revendications

1. Électrode sérigraphiée (1) permettant de détecter un polluant dans un échantillon d'eau comprenant :
- un substrat (2) ;
- une pluralité de pistes conductrices (3), sérigraphiées sur le substrat (2) ;
- une cellule électrochimique (4), connectée auxdites pistes conductrices (3) et conçue pour recevoir l'échantillon d'eau, ladite cellule électrochimique (4) comprenant en outre :
- une électrode de travail (5), sérigraphiée sur le substrat (2) et conçue pour permettre la dégradation électrochimique du polluant présent dans l'échantillon d'eau ;
- une électrode de pseudo-référence (6), sérigraphiée sur le substrat (2) et conçue pour fournir un potentiel électrique stable par rapport auquel le potentiel de l'électrode de travail (5) est défini ; et,
- une électrode auxiliaire (7), sérigraphiée sur le substrat (2) et conçue pour fournir une voie de passage pour la circulation d'un courant électrique dans la cellule électrochimique (4) ;
- une couche isolante (8), agencée sur les pistes conductrices (3) et adaptée de manière à protéger lesdites pistes conductrices (3) d'un environnement liquide ; et
- un élément filtrant (9), agencé en contact avec la cellule électrochimique (4) et conçu pour filtrer l'échantillon d'eau reçu par la cellule électrochimique (4) ;
et **caractérisée en ce que** l'élément filtrant (9) est imprégné d'un électrolyte.

2. Électrode sérigraphiée selon la revendication précédente, dans laquelle l'électrode de travail (5) et, en option, l'électrode auxiliaire (7) comprend/comprennent une encre à base de nanoparticules métalliques et de composite de carbone.

3. Électrode sérigraphiée selon la revendication précédente, dans laquelle l'encre à base de nanoparticules métalliques et de composite de carbone comprend un matériau en vrac de carbone, une pluralité de fibres de carbone et une pluralité de nanoparticules de cuivre.

4. Électrode sérigraphiée selon l'une quelconque des revendications précédentes, dans laquelle l'élément filtrant (9) imprégné d'électrolyte :
- comprend un matériau en papier poreux ; et/ou
- est recouvert d'une couche de fixation en plastique (10) contenant une pluralité de trous.

5. Électrode sérigraphiée selon l'une quelconque des revendications précédentes, dans laquelle l'électrolyte comprend de l'hydroxyde de sodium.

6. Capteur électrochimique permettant de mesurer la demande chimique en oxygène dans un échantillon d'eau contenant des matières organiques, **caractérisé en ce que** ledit système électrochimique comprend :
- une électrode sérigraphiée (1) selon l'une quelconque des revendications précédentes ;
- des moyens (11) permettant d'appliquer un potentiel électrique entre l'électrode de travail (5) et l'électrode de pseudo-référence (6) de ladite électrode sérigraphiée (1) ; et,
- des moyens (12) permettant de mesurer et enregistrer un courant faradique au niveau de ladite électrode de travail (5).

7. Capteur électrochimique selon la revendication précédente, dans lequel les moyens (11) d'application de potentiel et les moyens (12) de mesure et d'enregistrement de courant sont compris dans un potentiostat portatif alimenté et commandé par un dispositif mobile électronique (13).

8. Procédé de mesure de la demande chimique en oxygène dans un échantillon d'eau contenant des matières organiques au moyen du capteur électrochimique selon l'une quelconque des revendications 6 à 7, **caractérisé en ce que** ledit procédé comprend la réalisation des étapes suivantes :
- distribution de l'échantillon d'eau sur l'élément filtrant (9) imprégné d'électrolyte ;
- application d'un potentiel électrique entre l'électrode de travail (5) et l'électrode de référence pseudo-auxiliaire (6) par le moyen (11) d'application de potentiel ;
- mesure et enregistrement d'un courant faradique généré au niveau de l'électrode de travail (5) par le moyen (12) de mesure et d'enregistrement de courant ; et,
- détermination de la COD de l'échantillon d'eau à partir du courant faradique mesuré.

9. Procédé de fabrication d'une électrode sérigraphiée (1) selon la revendication 1, **caractérisé en ce que** ledit procédé comprend la réalisation des étapes suivantes dans un quelconque ordre techniquement possible :
- fourniture d'un substrat (2) ;
- sérigraphie d'une pluralité de pistes conductrices (3) et d'une électrode de pseudo-référence (6) sur le substrat (2) à l'aide d'un matériau électroconducteur ;
- sérigraphie d'une électrode de travail (5) et d'une électrode auxiliaire (7) sur le substrat (2) à l'aide d'une encre à base de nanoparticules métalliques et de composite de carbone ;
- sérigraphie d'une couche isolante (8) à l'aide d'une pâte diélectrique photodurcissable, et agencement de ladite couche isolante (8) sur les pistes conductrices (3) ;
- fourniture d'un élément filtrant (9) imprégné d'électrolyte ; et,
- agencement dudit élément filtrant (9) imprégné d'électrolyte en contact avec la cellule électrochimique (4).

10. Procédé de fabrication d'une électrode sérigraphiée (1) selon la revendication précédente, comprenant en outre l'étape d'imprégnation de l'élément filtrant (9) avec un électrolyte.

11. Procédé de fabrication d'une électrode sérigraphiée (1) selon l'une quelconque des revendications 9 à 10, dans lequel ledit procédé comprend en outre :
- la préparation d'une encre à base de nanoparticules de cuivre et de nanocomposite de carbone ; et,
- la sérigraphie de l'électrode de travail (5) et de l'électrode auxiliaire (7) sur le substrat (1) à l'aide de ladite encre à base de nanoparticules de cuivre et de nanocomposite de carbone.

12. Procédé de fabrication d'une électrode sérigraphiée (1) selon la revendication précédente, dans lequel l'étape de préparation d'une encre à base de nanoparticules de cuivre et de nanocomposite de carbone comprend en outre :
- la préparation d'un échantillon aqueux comprenant 30 à 35 % en poids de résorcinol, 0,2 à 0,6 % en poids de carbonate de sodium et 64 à 70 % en poids de formaldéhyde (échantillon A) ;
- la préparation d'un échantillon aqueux comprenant du nitrate de cuivre (II) hydraté à une concentration comprise entre 0,6 et 0,7 mol/L (échantillon B) ;
- le mélange des échantillons, A et B dans un rapport de volume A:B compris entre 3:1 et 3,5:1 pendant une période comprise entre 60 et 75 minutes (échantillon C) ;
- la dissolution de 450 à 600 mg de carbonate de sodium dans trois additions de 150 à 200 mg dans l'échantillon C et agitation pendant 1 à 1,5 heure jusqu'à ce que le pH soit compris entre 8 et 9 (échantillon D) ;
- le chauffage de l'échantillon D à une température comprise entre 55 et 65 °C pendant une période comprise entre 20 et 24 heures, résultant en une pluralité de gels humides :
- le placement des gels humides sous une hotte à température ambiante pendant au moins 2 jours ;
- la carbonisation de la poudre de nanoparticules de cuivre et de nanocomposite de carbone résultante sous un flux d'argon compris entre 80 et 120 cm³/min à une température comprise entre 1000 et 1055 °C pendant une période comprise entre 110 et 130 minutes ; et,
- le mélange de la poudre de nanoparticules de cuivre et de composite de carbone avec 15 à 20 % en poids de nitrocellulose préparée dans de l'acétate de 2-butoxyéthyle dans un rapport molaire en poids compris entre 3:1 et 3,5:1 jusqu'à ce que la pâte résultante présente une texture semblable à celle du miel.

13. Procédé de fabrication d'une électrode sérigraphiée (1) selon l'une quelconque des revendications 9 à 12, comprenant en outre :
- la fourniture d'une couche de fixation en plastique (10) contenant une pluralité de trous ; et
- la fixation de l'élément filtrant (9) imprégné d'électrolyte à la cellule électrochimique (4) au moyen de ladite couche de fixation en plastique (10).

14. Utilisation de l'électrode sérigraphiée (1) selon l'une quelconque des revendications 1 à 5 pour :
- la détermination de la demande chimique en oxygène dans les eaux de surface, les eaux usées et les déchets dangereux aqueux ; ou,
- la détection des ions halogénés, de la sucralose, ou des sous-produits chlorés de désinfection.
